(19) Europäisches Patentamt

European Patent Office

Office européen des brevets

(11) EP 0 896 887 A1

(12) **DEMANDE DE BREVET EUROPEEN**

(43) Date de publication:
17.02.1999 Bulletin 1999/07

(51) Int. Cl.⁶: **B60B 27/02**, B60B 1/04

(21) Numéro de dépôt: 98116781.0

(22) Date de dépôt: 31.10.1997

(84) Etats contractants désignés:
**BE CH DE ES GB IT LI NL**

(30) Priorité: **07.11.1996 FR 9613847**

(62) Numéro(s) de document de la (des) demande(s) initiale(s) en application de l'article 76 CBE:
**97118992.3 / 0 841 549**

(71) Demandeur: **MAVIC S.A.**
**01990 Saint-Trivier sur Moignans (FR)**

(72) Inventeurs:
• **Kubacsi, Michel**
**01600 Toussieux (FR)**

• **Mercat, Jean-Pierre**
**01990 Saint Trivier sur Moignans (FR)**

(74) Mandataire: **Lejeune, Benoit**
**Salomon S.A.**
**D.J.P.I.**
**74996 Annecy Cedex 09 (FR)**

Remarques:
Cette demande a été déposée le 05 - 09 - 1998 comme demande divisionnaire de la demande mentionnée sous le code INID 62.

(54) **Moyeu de roue de cycle et roue équipée d'un tel moyeu**

(57) L'invention concerne une roue de cycle comprenant un moyeu central (2) avec un arbre transversal (18) définissant un axe transversal (3), un corps de moyeu (10) monté en rotation libre autour de l'arbre (18), avec une partie médiane (11) et deux joues latérales (12, 13), un embout (15) prévu pour recevoir au moins un pignon de transmission, un mécanisme de roue libre unidirectionnelle, une jante (5), et deux nappes de rayons reliant la jante (4) à chacune des joues (12, 13) du moyeu.

La nappe de rayons (6) située du côté du pignon est formée par des rayons (6a, 6b) orientés de façon radiale ou quasi-radiale, et que les rayons de l'autre nappe de rayons (7) sont inclinés et croisés.

Fig. 2

Printed by Xerox (UK) Business Services
2.16.7/3.6

EP 0 896 887 A1

## Description

L'invention concerne un moyeu de roue de cycle équipé d'un mécanisme d'entraînement, notamment un moyeu arrière. L'invention concerne également une roue de cycle comprenant un tel moyeu.

Parmi les moyeux arrière, l'invention concerne plus particulièrement ceux qui permettent la mesure du couple moteur transmis par la chaîne, c'est-à-dire par le cycliste dans le cas d'une bicyclette.

Dans ce cas, un tel dispositif renseigne son utilisateur sur la puissance instantanée qu'il développe. Ceci peut être utile par exemple dans le cadre d'un programme d'entraînement de l'athlète pour doser les efforts aux moments voulus. Ceci peut être utilisé aussi en compétition, pour aider le coureur à se placer dans des conditions optimales de dépenses physiques. D'autres utilisations existent, par exemple pour des programmes de rééducation.

Il est courant de mesurer la puissance dépensée par un sportif ou par une personne en rééducation sur des ergomètres et des bicyclettes d'intérieur. Dans ce cas, on mesure simplement la puissance dissipée dans un organe de freinage ou autre.

Le problème est différent ici. En effet, une bicyclette est un engin mobile mu par la puissance développée par le cycliste. La mesure de puissance doit être faite sans dissipation ou perte d'énergie. Autrement les performances propres de la bicyclette elle-même seraient affectées.

Un moyeu permettant la mesure de couple est par exemple connu de la demande de brevet allemand publiée sous le numéro DE 31 50 149. Ce dispositif comprend un corps de moyeu supportant les rayons et la jante, et un tube central de transmission portant les pignons d'entraînement de la chaîne. Une liaison élastique relie ce tube central et le corps de moyeu, et un dispositif de lecture mesure le décalage angulaire entre les deux éléments. Ce dispositif pose des problèmes au niveau de la réalisation pratique. En effet, la liaison élastique apparaît comme l'élément principal de la mesure de couple. Il est difficile d'étalonner de façon fiable un tel élément, c'est-à-dire de trouver un élément dont l'élasticité est invariable dans le temps, et insensible aux conditions d'utilisation. En outre, la construction est relativement complexe car le corps de moyeu est monté en rotation libre sur le tube central de transmission, lui-même monté en rotation libre sur un arbre.

Un autre moyeu permettant la mesure du couple d'entraînement est connu par la demande de brevet européen publiée sous le numéro EP 0 393 427. Comme le moyeu précédent, ce moyeu comprend un corps de moyeu monté sur un tube central de transmission. Le tube central porte les pignons d'entraînement à l'une de ses extrémités, et son autre extrémité est reliée solidairement au corps de moyeu. Sous l'action du couple d'entraînement, le tube central de transmission se déforme en torsion. Comme l'angle de rotation relative entre les deux extrémités du tube est relativement faible, on amplifie ce décalage angulaire au moyen d'un petit levier monté basculant.

Une fois encore, la construction est relativement complexe, étant donné que le moyeu est monté en rotation libre par rapport au tube de transmission au moins du côté des pignons.

Du côté des pignons, l'isolation en rotation du tube de transmission et du moyeu est difficile à réaliser. A ce niveau, il existe également des problèmes d'étanchéité. A l'autre extrémité du tube de transmission, la liaison par encastrement du tube de transmission au moyeu est particulièrement délicate à réaliser. Aux deux extrémités, le moindre jeu de fonctionnement ou la moindre élasticité fausse la mesure angulaire et induit des erreurs significatives. Il apparaît également que le levier basculant réalise une amplification qui n'est pas linéaire.

Un but de l'invention est de proposer un moyeu permettant une mesure du couple d'entraînement dont la construction est plus simple. Un autre but de l'invention est de proposer un moyeu permettant une mesure plus fiable, c'est-à-dire diminuant les sources d'erreur, et présentant une linéarité améliorée.

Le moyeu de roue, notamment de roue de cycle, selon l'invention comprend un arbre transversal prévu pour être assemblé de façon solidaire sur un cadre, et définissant un axe transversal de révolution, un corps de moyeu monté en rotation libre autour de l'arbre transversal, le corps de moyeu ayant une partie médiane, deux joues latérales, les joues latérales ayant des moyens d'accrochage prévus pour des moyens de liaison avec une jante, le corps de moyeu ayant au-delà de l'une des joues un embout latéral sur lequel est monté un mécanisme de roue libre prévu pour recevoir au moins un pignon d'entraînement.

Le moyeu est caractérisé par le fait que la partie médiane du corps de moyeu est conformée en corps d'épreuve déformable en torsion autour de l'axe transversal de révolution, et un couple de deux capteurs est disposé dans le moyeu, les capteurs étant prévus pour mesurer au moins de façon séquentielle le décalage angulaire entre les deux extrémités du moyeu par rapport à l'axe transversal de révolution.

La roue de cycle selon l'invention comprend un moyeu central avec un arbre transversal définissant un axe transversal, un corps de moyeu monté en rotation libre autour de l'arbre, avec une partie médiane et deux joues latérales et un embout prévu pour recevoir au moins un pignon de transmission et un mécanisme de roue libre unidirectionnelle, une jante, et deux nappes de rayons reliant la jante à chacune des joues du moyeu.

Elle est caractérisée par le fait que la partie médiane du corps de moyeu est conformée comme un corps d'épreuve déformable en torsion, que les nappes de rayons sont prévues pour que la nappe de rayons située du côté opposé à l'embout soit destinée à transmettre une partie déterminée du couple d'entraînement transmis à l'embout par le pignon et le mécanisme de roue libre, et que le moyeu présente des capteurs prévus pour mesurer la position angulaire relative de chacune de ses joues.

Ainsi, dans le cas présent, c'est le corps de moyeu lui-même dont on mesure la déformation en torsion. De plus, on favorise cette torsion en maîtrisant entre les deux nappes de rayons la répartition du couple d'entraînement qui est transmis à la jante et au pneu.

D'habitude en effet, c'est la nappe de rayons située à proximité des pignons qui transmet la majeure partie de ce couple. Ici, au contraire, une partie, et, de préférence la majeure partie, du couple est transmis par la nappe de rayons opposée aux pignons. Cette partie traverse le corps de moyeu et génère une torsion qui varie avec le couple transmis.

L'invention sera mieux comprise en se référant à la description ci-dessous et aux dessins en annexe qui en font partie intégrante.

La figure 1 représente une roue arrière de cycle vue de face.
La figure 2 est la roue de la figure 1 vue du côté des pignons.
La figure 3 est la roue de la figure 1 vue du côté opposé aux pignons.
La figure 4 représente en vue de face le moyeu de la roue selon un mode particulier de mise en oeuvre de l'invention.
La figure 5 représente en coupe le corps du moyeu de la figure précédente.
La figure 6 est une vue en coupe du moyeu selon le plan DD repéré en figure 5.
La figure 7 est une vue en coupe du corps de moyeu selon la vue HH repérée en figure 5.
La figure 8 est une vue du corps de moyeu selon la coupe FF repéré en figure 5.
La figure 9 est une vue semblable à la figure 8 qui illustre le fonctionnement du dispositif multiplicateur en charge.
La figure 10 représente en une vue de côté, de façon agrandie, la came du capteur associée aux moyens multiplicateur.

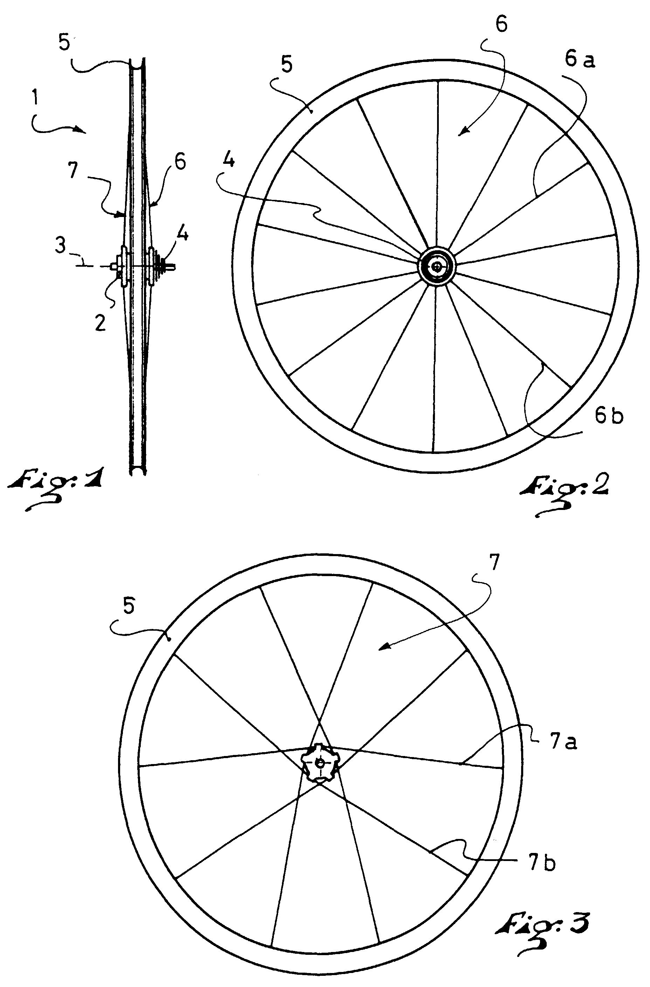

La figure 1 représente à titre d'illustration de l'invention une roue 1. Cette roue comprend un moyeu central 2 définissant un axe de révolution transversal 3.

D'un côté du moyeu se trouve un boîtier 4 de pignons qui sont prévus pour servir de renvoi à une chaîne d'entraînement. La roue comprend par ailleurs une jante annulaire 5 centrée sur l'axe 3. La jante est reliée aux deux extrémités du moyeu par deux nappes de rayons, respectivement 6 et 7.

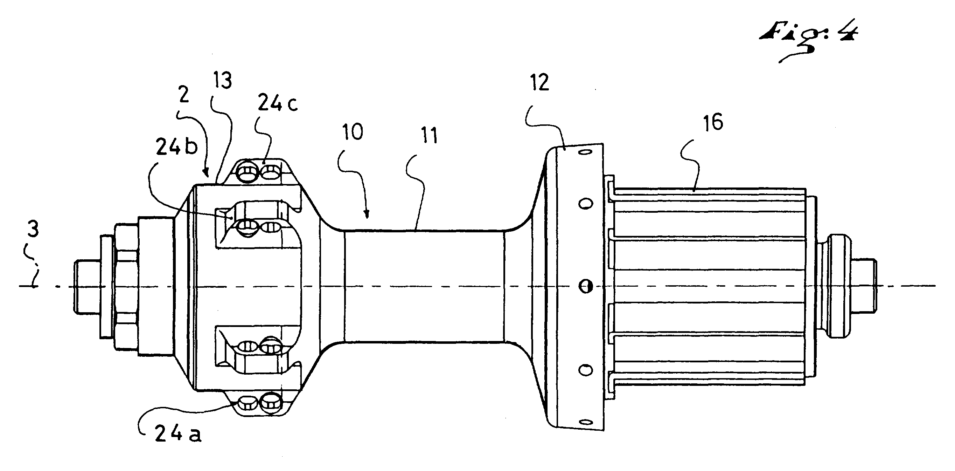

La figure 2 représente au premier plan les rayons 6a, 6b, ... de la nappe de rayons 6 située du côté du boîtier 4 de pignons. La figure 3 représente l'autre côté de la roue, avec en premier plan les rayons 7a, 7b,

.. de la nappe de rayons 7.

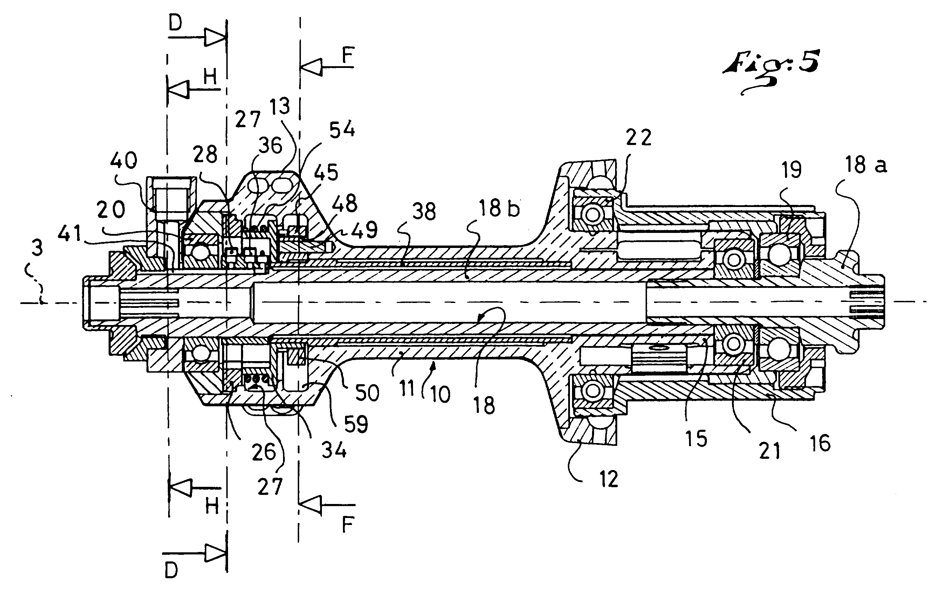

En se référant aux figures 4 et 5, le moyeu 2 comprend un corps de moyeu 10. Le corps de moyeu 10 présente approximativement la forme d'un diabolo centré sur l'axe 3. Il présente une partie médiane 11 de diamètre relativement petit, et deux joues latérales ou renflements 12 et 13 de diamètre supérieur. Ces joues sont prévues pour fournir des points d'ancrage aux différents rayons des nappes.

A l'une de ses extrémités, le corps de moyeu présente un embout 15 destiné à recevoir un corps de roue libre 16. Extérieurement, le corps de roue libre présente une surface cylindrique cannelée qui est prévue pour recevoir le boîtier de pignons. Un mécanisme de roue libre unidirectionnelle assure la liaison entre le corps de moyeu et le corps de roue libre. Un tel mécanisme est connu, par exemple d'après la demande de brevet publiée sous le numéro EP 0 703 376, et ne sera pas décrit en détail. A ce niveau, tout mécanisme approprié de roue libre peut convenir. On pourrait également construire le corps de moyeu d'une autre façon, afin qu'il soit prévu pour recevoir un mécanisme de roue libre indépendant, qui est rapporté à l'une de ses extrémités. Ces différents montages sont connus de l'homme du métier.

Le corps de moyeu et le corps de roue libre sont montés libres en rotation autour d'un arbre transversal 18 centré sur l'axe 3. L'arbre 18 est prévu pour être monté de façon solidaire sur un cadre de cycle. Par exemple, l'arbre 18 est creux, et l'assemblage est réalisé par une tige de blocage rapide d'un type connu. Des roulements 19, 20 sont situés à chacune des extrémités de l'arbre, et entre le corps de moyeu et l'arbre. D'autres roulements 21, 22 se trouvent entre le corps de roue libre, l'arbre transversal et le corps de moyeu.

L'arbre transversal 18 est ici en deux parties 18a et 18b, assemblées l'une à l'autre par des parties correspondantes taraudées et filetées. Des rondelles épaulées sont prévues aux extrémités des parties 18a, et 18b pour prendre appui contre les roulements externes. Les deux parties 18a, 18b sont vissées et bloquées. Un écrou comprenant l'une des rondelles épaulées est monté sur l'une des parties à son extrémité, et permet de régler le jeu de fonctionnement par son serrage . Des rondelles d'étanchéité ou tout autre moyen approprié est par ailleurs prévu à chacune des ouvertures du corps de moyeu.

Selon l'invention, le corps de moyeu est conformé en corps d'épreuve déformable en torsion autour de l'axe 3 sous l'action du couple d'entraînement que la chaîne transmet au pignon en prise et au corps de roue libre. En outre, des capteurs sont prévus pour mesurer le décalage angulaire entre les deux extrémités du corps de moyeu.

En se référant aux figures 4 et 5, la partie médiane 10 du corps de moyeu présente un diamètre externe relativement petit, et une épaisseur de paroi relativement fine. En outre, le corps de moyeu est réalisé en un matériau tel qu'un alliage d'aluminium. On a obtenu de bons résultats avec un alliage d'aluminium connu sous la référence 2014 T6 ou 7075 T6. Ces matériaux présentent une bonne élasticité, une limite élastique élevée. De plus, un alliage d'aluminium a l'avantage d'être léger. Naturellement tout autre matériau approprié convient.

On a obtenu de bons résultats avec une partie médiane présentant une longueur de 35 à 40 millimètres environ, un diamètre externe de 20 millimètres environ, et une épaisseur de paroi de 2 millimètres environ. Naturellement ces chiffres n'ont qu'une valeur indicative. De préférence, à la jonction entre les extrémités de la partie médiane et les joues, le corps de moyeu présente un rayon relativement important, ou une variation progressive de diamètre. Ceci évite les zones de rupture.

Pour solliciter le corps de moyeu à la torsion, les deux nappes de rayons sont prévues pour transmettre le couple d'entraînement du moyeu à la jante selon une répartition déterminée, qui, de préférence, ne varie pas avec l'intensité du couple ou la tension des rayons.

Selon le mode de répartition adopté pour le mode de réalisation illustré, la majeure partie du couple est transmis à la jante par la nappe de rayons 7 située du côté opposé aux pignons. Ainsi, cette partie du couple traverse le corps de moyeu, et génère une torsion qui est mesurée par des capteurs.

Pour ce faire, comme cela est visible dans la figure 2, la nappe de rayons 6 située du côté des pignons est formée par des rayons 6a, 6b orientés de façon radiale, ou quasi radiale compte tenu de l'inclinaison des rayons en direction du plan défini par la jante. La joue 12 prévue pour l'accrochage de ces rayons présente la forme d'une coupelle ouverte du côté de l'embout 15. La coupelle peut être une pièce indépendante rapportée sur le corps de moyeu et indexée, c'est-à-dire immobilisée dans une position angulaire précise, par exemple par une goupille, ou par un encastrement. La paroi de la coupelle est percée d'un pluralité d'orifices dans lesquels les rayons sont enfilés pour être retenus par leur tête. Les rayons utilisés ici sont des rayons de type droit, c'est-à-dire avec la tête dans le prolongement de la tige. Ces rayons sont réputés pour supporter une tension élevée. On a obtenu de bons résultats avec une nappe 6 formé de dix rayons radiaux.

Un tel mode de rayonnage est adapté pour assurer la liaison entre la jante et le moyeu. Par contre, compte tenu de l'orientation radiale des rayons, il n'est pas adapté pour transmettre un couple d'entraînement.

A l'inverse, la nappe 7 présente un rayonnage croisé. Les rayons 7a, 7b, de la nappe 7 sont inclinés par rapport à une direction radiale, pour favoriser la transmission du couple vers la jante. Ceci est visible dans la figure 3.

En se référant aux figures 4 et 5, la joue 13 présente une pluralité d'oreilles 24a, 24b, 24c...orientées de façon radiale. Chacune des oreilles présente deux orifices dans lesquels deux rayons sont enfilés tête-bêche pour être retenus par leur tête respective. Les rayons sont également des rayons droits. On a obtenu de bons résultats avec une nappe 7 formée de dix rayons 7a, 7b, .... reliés à cinq oreilles 24a, 24b ....

Naturellement, ces chiffres ne sont donnés qu'à titre indicatif, et le nombre de rayons de chacune des nappes pourrait être plus ou moins élevé selon l'utilisation prévue de la roue, notamment selon qu'il s'agit d'une roue pour un vélo de route ou un vélo tout terrain.

Pour la roue qui vient d'être décrite, le couple d'entraînement se répartit globalement de la façon suivante, 95% environ du couple est transmis par la nappe 7, et le reste, soit 5% environ par la nappe 6. La partie du couple transmise par la nappe 6 n'est pas exactement linéaire avec l'intensité du couple, à cause de l'augmentation de tension des rayons qui se produit pour des couples élevés. La répartition dépend également de la tension initiale des rayons. Toutefois, cette non-linéarité est peu marquée compte tenu du mode de rayonnage adopté. A ce sujet, on a remarqué qu'un rayonnage croisé des deux nappes présentait une sensibilité plus élevée à la tension initiale des rayons.

En outre la proportion du couple transmis par la nappe 6 est très faible, si bien que l'on peut considérer globalement que cette répartition est constante sur une plage de couple d'entraînement correspondant à l'utilisation normale d'une bicyclette. Ainsi, on peut considérer que le corps de moyeu est soumis à un couple de torsion proportionnel au couple d'entraînement. A titre indicatif, on a estimé que dans cette plage, le couple pouvait varier entre 0 et 250 N.m (Newtons-mètres) environ. A titre indicatif, pour une telle plage de valeurs, la torsion du corps de moyeu peut varier entre zéro et deux degrés et demi à trois degrés d'angle mesuré entre les deux extrémités du corps de moyeu, c'est-à-dire au niveau des deux joues 12 et 13.

Selon une variante de réalisation, la coupelle présente une couronne indépendante à laquelle les rayons de la nappe 6 sont accrochés et qui peut pivoter librement par rapport au reste du corps de moyeu. De préférence, dans ce cas, cette couronne indépendante est reliée au corps de moyeu par un roulement ou autre moyen similaire assurant une libre rotation. Ce mode de construction est adapté par exemple pour une roue à disques pleins, où on ne peut pas maîtriser la répartition du couple entre les deux nappes par le mode de rayonnage, pour la simple raison que ce sont les disques et non des rayons qui relient le moyeu et la jante.

De cette façon, on augmente encore la proportion du couple qui est transmise à la nappe 7 par le corps de moyeu.

Selon ce qui vient d'être décrit, on comprend que l'on recherche à transmettre par le corps de moyeu la plus grande partie du couple. Ceci n'est pas limitatif, et toute répartition pourrait convenir, avec une partie du couple plus ou moins importante transmise par la nappe 6, pourvu que la nappe de rayons 7 transmette au moins une partie non nulle de ce couple, cette partie traversant le corps de moyeu 12.

Autant que possible, la répartition est constante sur la plage de couple considéré, c'est-à-dire que le corps de moyeu est soumis à un couple de torsion proportionnel au couple d'entraînement. Selon une autre possibilité, on adopte une répartition donnée à priori non-linéaire, on étalonne pour cette répartition l'angle de torsion par rapport au couple d'entraînement, et on corrige la non-linéarité par un traitement numérique, au niveau d'une unité de traitement qui sera décrite plus loin.

L'angle de torsion entre les deux extrémités du corps de moyeu, globalement entre les deux joues 12 et 13, est mesuré au moyen de deux capteurs reliés à chacune des extrémités. Chacun des capteurs comprend une came en forme de bague, qui est liée en rotation avec l'extrémité du corps de moyeu à laquelle elle est associée, et un rupteur fixe monté sur l'arbre transversal. Ainsi, la figure 6 représente une came annulaire 26 montée par encastrement dans un logement 27 situé à l'intérieur du corps de moyeu, au niveau de sa joue 13. En direction de l'arbre transversal 18, la came présente une pluralité de dents 26a, 26b... qui sont réparties de façon régulière à la surface interne de la came. Un rupteur 28 est monté sur l'arbre 18 en regard de la came 26. Ce rupteur comprend une lamelle métallique 29 dont une extrémité libre est en appui contre un plot conducteur 30. Au-delà du plot, la lamelle présente une extrémité en forme d'anse 31 qui s'étend en direction des dents de la came. A chaque passage d'une dent, l'anse est soulevée, ce qui coupe la liaison électrique entre la lamelle et le plot. Après le passage de la dent, le contact électrique est rétabli entre la lamelle et le plot.

Un capteur semblable est associé à l'autre extrémité du corps de moyeu. Ce capteur présente une came 34 de même nature que la came 26, présentant des dents identiques réparties de façon identique. La figure 6 représente les dents 35a, 35b ... de la came 34. Un rupteur 36 semblable au rupteur 28 est par ailleurs monté sur l'arbre 18 en regard de la came 34. De préférence, pour un couple nul, les deux cames sont calées par rapport à leur rupteur respectif de façon qu'il existe un léger décalage initial entre l'ouverture des deux rupteurs. La mesure de torsion du corps de moyeu consiste à mesurer la variation de ce décalage au passage de chacune des dents sur le rupteur. Le nombre de dents de chaque came détermine le nombre de fois où la mesure est réalisée au cours d'un tour complet. La figure 6 montre huit dents, ce nombre n'est cependant pas limitatif et tout nombre inférieur ou supérieur convient. Ainsi que cela est visible dans la figure 5, de préférence, les deux capteurs sont côte à côte. Ils sont situés du côté opposé aux pignons dans le logement 27 situé à l'extrémité du corps de moyeu, au niveau de la joue 13. Un tube 38 ramène par ailleurs de ce côté l'information sur la position angulaire de l'autre extrémité du corps de moyeu. Le tube 38 traverse le corps de moyeu sur une grande partie de sa longueur. Il s'étend entre l'arbre 18 et la partie médiane 11 du corps de moyeu. Du côté de la joue 12, il présente une extrémité encastrée dans le corps de moyeu. De préférence, l'encastrement est réalisé par un collage, ou toute autre technique appropriée qui permet de caler angulairement le tube 38 autour de l'axe 3. Ceci permet de régler le déphasage initial à une valeur déterminée. Le tube 38 ne transmet quasiment aucun effort. Il présente de ce fait une paroi très fine, et il est réalisé en un matériau léger, par exemple un alliage d'aluminium, de préférence dans un alliage connu sous le nom de "ZAMAC". Cet alliage de zing, d'aluminium et de manganèse, présente l'avantage d'avoir un module élastique supérieur à celui d'un alliage d'aluminium. Les cames sont quant à elles réalisées en une matière plastique.

Ainsi, contrairement aux dispositifs connus, c'est ici le corps de moyeu qui est le corps d'épreuve soumis à une torsion. Le tube 38 ne fait que ramener une information angulaire vers une extrémité du moyeu.

Des fils électriques de connexion non représentés dans les figures relient par ailleurs les lamelles et les plots des rupteurs à une prise de raccordement qui est logé dans le canon 40 situé dans la partie extrême de l'arbre central.

La figure 7 représente une section transversale du moyeu à ce niveau. Le canon est porté par un élément 41 rapporté, et assemblé au reste du corps de moyeu avec l'assemblage des deux parties 18a et 18b de l'arbre 18. Une rainure 41 pour le passage des fils est visible dans cette figure.

L'angle de torsion entre les deux extrémités du corps de moyeu étant relativement faible, l'invention propose de mesurer cet angle au travers de moyens multiplicateur qui vont maintenant être décrits. Dans le cas présent ces moyens sont interposés entre la joue 12, par l'intermédiaire du tube 38, et la came 34 qui lui est associée.

Les moyens multiplicateur comprennent une première portion de roue dentée 44 portée par un élément central 45 assemblé à l'extrémité libre du tube 38, ou réalisés de façon monobloc avec le tube. La portion 44 est orientée vers l'axe 3, elle est circulaire et centrée sur cet axe.

La portion 44 est en prise avec la denture 48a d'un pignon de petit diamètre 48 montée pivotante autour d'un axe 49. L'axe est porté par l'extrémité du corps de moyeu situé du côté de la joue 13, c'est-à-dire en quelque sorte, l'extrémité de référence de la mesure par rapport à laquelle le déphasage angulaire de l'autre extrémité est mesuré. L'axe 49 tourne autour de l'axe 3 avec la rotation de la roue.

Le pignon 48 est solidaire d'une sorte de balancier 50 qui s'étend depuis la zone de l'axe 49, jusqu'au delà de l'axe transversal 3. Le balancier 50 pivote autour de l'axe 3 avec la roue autour de l'arbre transversal, et autour de l'axe 49 avec la rotation du pignon 48, c'est-à-dire avec la torsion du corps de moyeu.

Le balancier 50 porte du côté opposé au pignon 48 une portion de roue dentée 51 orientée en direction de l'axe 3. Cette portion est en prise avec une autre portion 52 qui est située à la périphérie externe de la came 34. Comme cela est visible dans la figure 10, la portion 52 de la came 34 est déportée en direction de l'autre extrémité du moyeu. Ainsi, la rotation du balancier 50 autour de l'axe 49 entraîne à son tour la came 34 en rotation autour de l'arbre transversal 18. Un ressort cylindrique de torsion 54 exerce sur la came 34 un moment de rappel élastique en direction de la position initiale de la came. Ce ressort assure le rappel élastique de la came, ainsi que le rattrapage des jeux.

L'angle de torsion du corps de moyeu est ainsi multiplié dans un rapport lié au rapport des rayons des différentes portions de roue dentée, et de la position relative des axes de rotation. A titre indicatif, on a réussi de cette façon à atteindre un rapport de multiplication d'une valeur de "treize".

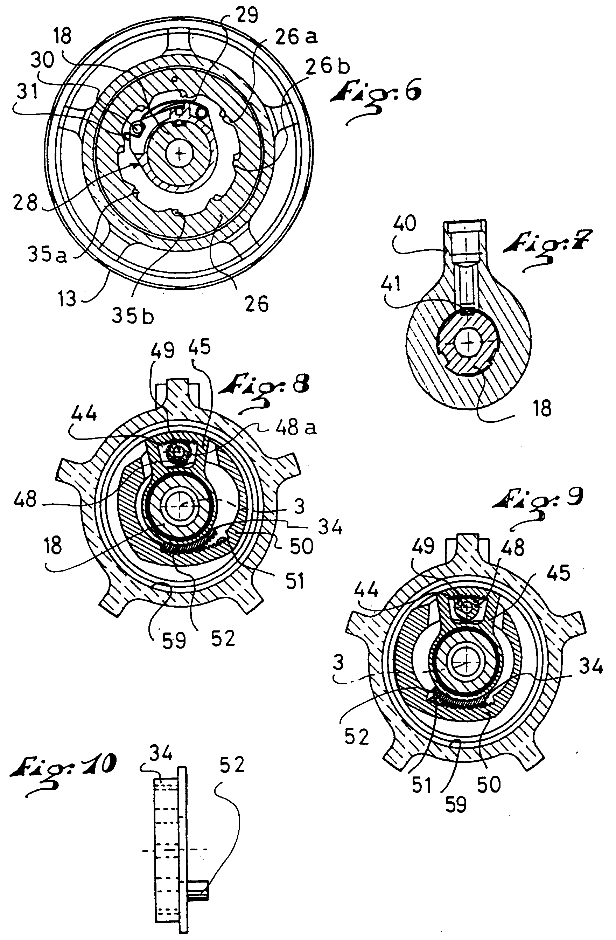

En se référant aux figures, la figure 8 représente la position relative des moyens multiplicateur pour un couple nul, et la figure 9 représente ces mêmes moyens pour un couple de l'ordre de 240 N.m. Comme cela est visible dans la figure 5, la came 34 et la came 26 sont en appui chacune des nervures annulaires qui délimitent des chambres successives pour les cames et les moyens multiplicateur. La chambre 59 en particulier est prévue pour permettre le débattement angulaire du balancier 50 autour de l'axe 49 sur toute la plage de mesure du couple de torsion.

Les différents éléments des moyens multiplicateur sont réalisés en tout matériau approprié. Avantageusement, ils sont réalisés en un alliage connu sous le nom commercial de ZAMAC qui est réputé pour réaliser par moulage des pièces d'une grande précision, et pour présenter un module élastique élevé.

Le moyeu qui vient d'être décrit est prévu pour être connecté à une unité de traitement. L'ouverture et la fermeture successive des rupteurs génèrent des impulsions électriques. Ces impulsions renseignent sur la vitesse pure de rotation de la roue par leur vitesse de succession, et sur le couple d'entraînement exercé par la chaîne, par leur déphasage.

L'unité de traitement est en mesure de calculer la puissance développée par le cycliste en traitant la mesure de torsion par rapport au temps. Cette unité de traitement comprend notamment des moyens de lissage des signaux recueillis par les capteurs. Le couple est en effet susceptible de varier de façon significative au cours d'un tour de pédalier. Au niveau de l'unité de traitement, la puissance instantanée est mesurée d'après la formule $P = C\omega$, où P désigne la puissance en watts, C le couple en N.m, et où la vitesse angulaire en rd/s (radian par seconde). La vitesse angulaire est directement déductible de la mesure du couple. Il suffit de mesurer l'écart de temps entre le passage de deux cames successives de l'un des capteurs. L'énergie développée par le cycliste est mesurée en intégrant la mesure de puissance par rapport au temps, c est-à-dire en appliquant la formule $E = \int Pdt$, où E est l'énergie en watt/seconde.

L'unité comprend par ailleurs tout moyen de traitement approprié, par exemple d'affichage instantané, d'affichage d'une valeur moyenne, de comparaison avec une valeur de consigne, de mémorisation de valeurs de consigne, d'enregistrement de la mesure de couple, de restitution des valeurs enregistrées. Eventuellement, tel que cela a été évoqué plus haut, l'unité de traitement peut présenter un filtre, notamment un filtre numérique pour corriger une non-linéarité éventuelle de la mesure de couple.

L'unité de traitement peut être combinée aussi à une unité générale de gestion électronique des performances du cycle et du cycliste.

Naturellement, la construction qui vient d'être décrite est susceptible d'être modifiée. En particulier, la position des capteurs du côté opposé à l'embout est avantageuse pour avoir une connexion électrique située du coté opposé au dérailleur arrière. Cette positon n'est pas limitative, et les capteurs pourraient être situés du côté de l'embout 15.

Les capteurs pourraient également être disposés vers chacune des extrémités du moyeu.

La liaison entre le moyeu et la jante par des rayons n'est pas limitative, et tout autre moyen approprié convient, par exemple des bâtons, des disques.

D'autres moyens pourraient aussi convenir pour multiplier la valeur du décalage angulaire entre les deux extrémités du moyeu.

Enfin, l'invention n'est pas limitée à une roue à deux nappes de rayons. Elle concerne également les roues où la liaison entre le moyeu et la jante comprend des disques, des bâtons ou une partie centrale en bulbe ou lenticulaire. Comme cela a été évoqué plus haut, dans ce cas, il n'est pas possible à priori de maîtriser la répartition de couple entre les deux côtés du moyeu.

Pour remédier à cela, du côté des pignons, une solution consiste à isoler par un roulement, ou autre moyen similaire, le moyeu et le reste de la roue, si bien que l'intégralité ou la quasi-intégralité du couple traverse le moyeu pour atteindre la jante.

**Revendications**

1. Roue de cycle comprenant un moyeu central (2) avec un arbre transversal (18) définissant un axe transversal (3), un corps de moyeu (10) monté en rotation libre autour de l'arbre (18), avec une partie médiane (11) et deux joues latérales (12, 13), un embout (15) prévu pour recevoir au moins un pignon de transmission, un mécanisme de roue libre unidirectionnelle, une jante (5), et deux nappes de rayons reliant la jante (5) à chacune des joues (12, 13) du moyeu, caractérisée par le fait que la nappe de rayons (6) située du côté du pignon est formée par des rayons (6a, 6b) orientés de façon radiale ou quasi-radiale, et que les rayons de l'autre nappe de rayons (7) sont inclinés et croisés.

2. Roue selon la revendications 1, caractérisée par le fait que les rayons (6a, 6b) de la nappe de rayons (6) sont des rayons droits.

3. Roue selon la revendication 1, caractérisée par le fait que les rayons (7a, 7b) de la nappe de rayons (7) sont des rayons droits.

4. Roue selon la revendication 3, caractérisée par le fait que la joue (13) qui reçoit les rayons (7a, 7b) de la nappe (7) présente des oreilles (24a, 24b, 24c) orientées de façon radiale, et que chacune des oreilles présente deux orifices dans lesquels les rayons sont enfilés tête-bêche.

5. Roue selon la revendication 1, caractérisée par le fait que chacune des nappes (6, 7) de rayons présente dix rayons.

6. Roue selon la revendication 1, caractérisée par le fait que le corps de moyeu (10) est déformable en torsion.

*Fig.1*

*Fig.2*

*Fig.3*

Fig. 4

Fig: 5

EP 0 896 887 A1

*Fig.6*

*Fig.7*

*Fig.8*

*Fig.9*

*Fig.10*

Office européen
des brevets

**RAPPORT DE RECHERCHE EUROPEENNE**

Numéro de la demande

EP 98 11 6781

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,P | FR 2 736 869 A (FRULLANI GUY) 24 janvier 1997 * page 5, ligne 30 - page 9, colonne 32; figures 4,5 * | 1-3,5 | B60B27/02 B60B1/04 |
| D,A | DE 31 50 149 A (SACHS SYSTEMTECHNIK) 30 juin 1983 * page 1 - page 3; figures * | 1 | |
| D,A | EP 0 393 427 A (LOOK) 24 octobre 1990 * abrégé; figures * | 1,5 | |
| D,A | EP 0 703 376 A (MAVIC) 27 mars 1996 * abrégé; figures * | 1 | |
| A | US 5 429 421 A (WATSON) 4 juillet 1995 * colonne 3, ligne 16 - colonne 5, ligne 38; figures * | 1 | |

DOMAINES TECHNIQUES
RECHERCHES (Int.Cl.6)

B60B

Le présent rapport a été établi pour toutes les revendications

| Lieu de la recherche | Date d'achèvement de la recherche | Examinateur |
|---|---|---|
| LA HAYE | 2 décembre 1998 | Vanneste, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)